# EUROPEAN PATENT SPECIFICATION

(11) **EP 4 065 966 B1**
(45) Date of publication and mention of the grant of the patent: **22.10.2025**
(21) Application number: 20811334.0
(22) Date of filing: 25.11.2020
(51) Int. Cl.: G01N 21/84

(54) **METHODS AND DEVICES FOR PERFORMING AN ANALYTICAL MEASUREMENT**
VERFAHREN ZUR DURCHFÜHRUNG EINER ANALYTISCHEN MESSUNG
PROCÉDÉ DE RÉALISATION D'UNE MESURE ANALYTIQUE

(30) Priority: 26.11.2019 EP 19211520
(43) Date of publication of application: 05.10.2022
(73) Proprietor: F. Hoffmann-La Roche AG, 4070 Basel (CH); Roche Diabetes Care GmbH, 68305 Mannheim (DE)
(72) Inventor: ALPEROWITZ, Lukas, 80805 München (DE); BERG, Max, 68305 Mannheim (DE); HAILER, Fredrik, 68305 Mannheim (DE); LIMBURG, Bernhard, 68305 Mannheim (DE); SELLMAIR, Sebastian, 80805 München (DE)
(74) Representative: Meinel, Anne Julia
(86) International application number: PCT/EP2020/083385
(87) International publication number: WO 2021/105223

(56) References cited:
- EP-A1- 1 801 568
- WO-A1-2012/131386
- DE-A1- 102016 202 428
- US-A1- 2018 024 049

## Description

### Technical Field

The present invention refers to a method of performing an analytical measurement based on a color formation reaction in an optical test strip by using a mobile device having a camera. The invention further relates to a computer program and a computer-readable storage medium with program means for executing the method according to the invention. Further, the invention refers to a mobile device and a kit for performing an analytical measurement. Methods, computer programs, mobile devices and kits according to the present invention may be used in medical diagnostics, in order to, for example, qualitatively detect one or more analytes in one or more body fluids. Other fields of application of the present invention, however, are feasible.

### Background art

In the field of medical diagnostics, in many cases, one or more analytes have to be detected in samples of body fluid, such as blood, interstitial fluid, urine, saliva or other types of bodily fluids. Examples of analytes to be detected are glucose, triglycerides, lactate cholesterol or other tapes of analytes typically present in these bodily fluids. According to the concentration and/or the presence of the analyte, an appropriate treatment may be chosen, if necessary. Without narrowing the scope, the invention specifically may be described with respect to blood glucose measurements. It shall be noted, however, that the present invention may be used for other types of analytical measurements using test elements.

Generally, devices and methods known to the skilled person make use of test elements comprising one or more test chemicals, which, in presence of the analyte to be detected, are capable of performing one or more detectable detection reactions, such as optically detectable detection reactions. As an example, EP 0 821 234 A2 describes a diagnostic test carrier for the determination of an analyte from whole blood with the aid of a reagent system contained in the carrier and a method for the determination of an analyte from whole blood with the aid of the diagnostic test carrier. The diagnostic test carrier includes a color forming reagent. The test field has a sample application side to which the blood sample is delivered and a detection side where an optically detectable change occurs as a result of the reaction of the analyte with the reagent system. Furthermore, the test field is designed so that the erythrocytes contained in the sample do not reach the detection side. In addition, the test field comprises a transparent film and a first and a second superposed film layer applied thereto, wherein the first layer on the transparent film is substantially less light-scattering in the wet state than the overlying second layer.

With regard to the test chemicals comprised in test elements, reference may be made e.g. to J. Hoenes et al.: The Technology Behind Glucose Meters: Test Strips, Diabetes Technology & Therapeutics, Volume 10, Supplement 1, 2008, S-10 to S-26. Other types of test chemistry are possible and may be used for performing the present invention.

In analytical measurements, specifically analytical measurements based on color formation reactions, one technical challenge resides in the evaluation of the color change which is due to the detection reaction. Besides using dedicated analytical devices, such as handheld blood glucose meters, the use of generally available electronics such as smart phones and portable computers has become more and more popular over the recent years. As opposed to measurements performed by using dedicated analytical measurement devices, when using mobile computing devices, such as smart phones, various influences need to be taken into account. As an example, lighting conditions, positioning, vibrations or other more or less uncontrollable conditions are to be considered. In the field of technology of mobile computing devices, various technical approaches have been developed over the recent years in order to improve image recognition and/or to gain additional information regarding, for example, unknown geometrical parameters of the setup.

Thus, as an example, US 2014/0170757 A1 describes a method for a portable computing device to read a reaction area on a test strip, which is located in a peripheral device placed over an image sensor and a light source of the portable computing device. Light is provided with the light source, which the peripheral device directs to the reaction area. An image including the reaction area is captured with the image sensor. An analyte characteristic is determined based on a color of the captured reaction area in the image.

Further, WO 2018/115346 A1 describes a system for capturing measurement images of an object to be measured, comprising a mobile electronic device, wherein the mobile electronic device comprises: a housing; a camera, integrated into the housing, for recording measurement images of an object to be measured within an observation region of the camera; a screen, integrated into the housing, for displaying images in a light-emitting manner, wherein the screen faces the observation region of the camera; a control unit, integrated into the housing, said control unit being configured to actuate the screen of the mobile electronic device to display a plurality of different illumination images of a predefined illumination image sequence, wherein the control unit is configured to actuate the camera of the mobile electronic device to capture one measurement image of the object to be measured in each case synchronously with displaying each illumination image of the predefined illumination image sequence. The invention moreover relates to a corresponding method and computer program product.

US 9,886,750 B2 describes an electronic device for reading diagnostic test results and collecting subject data for inclusion in a local chain of evidence database and for transferring and receiving data from remote databases.

Further, US 9,322,767 B2 describes devices and methods for performing a point of care blood, cell, and/or pathogen count or a similar blood test. Disclosed are systems that can be used to provide rapid, accurate, affordable laboratory-quality testing at the point of care. The systems described are capable of imaging and counting individual cells in a prepared cell sample (e.g., a peripheral blood smear or a blood sample prepared in a microfluidic device) or another prepared cell-containing sample without the need for a microscope or other expensive and cumbersome optics. The systems described are designed to eliminate or replace expensive, centralized clinical testing equipment and technical personnel. Such systems may include automated data reporting and decision support.

US 2014/0005498 A1 describes a method including a camera of a mobile electronic device capturing a photo of at least one eye of a patient, a photo of a finger of the patient, and a photo of at least one type of medication taken by the patient. The method can also include administering a motor test to the patient and storing in a database results of the motor test along with the captured photos.

Further, software Apps for use with a smart phone are available for download, such as the ACCU-CHEK^{®} SugarView App by Roche Diabetes Care GmbH, Germany, available under https://www.accu-chek-sugarview.com.

US 2018/024049 A1 describes a method and a calorimetric device for performing colorimetric analysis of a test fluid to evaluate associated physiological parameters. The images of the test strip at different heights are captured by the calorimetric device and based on analysis of the captured images, a plurality of geometric parameters respectively associated with the test strip is determined. Based on the plurality of geometric parameters, an image resizing factor is determined and resized images are generated based on the image resizing factor. Upon generating the resized images, calorimetric values respectively associated with the resized images are determined based on which physiological parameters associated with the test fluid are evaluated.

WO 2012/131386 A1 describes a testing apparatus for performing an assay, the testing apparatus comprising: a receptacle containing a reagent, the reagent being reactive to an applied test sample by developing a color or pattern variation; a portable device, e.g. a mobile phone or a laptop, comprising a processor and an image capture device, wherein the processor is configured to process data captured by the image capture device and output a test result for the applied test sample.

EP 1 801 568 A1 describes a method which involves positioning a camera at a test strip for pictorially detecting a color indicator and a reference color area. A measured value is determined for the relative position between the camera and the strip and compared with a desired value area. The camera is moved to reduce deflection relative to the strip during the deflection between the measured value and the desired value. An image area assigned to the indicator is localized in a colored image that is detected by the camera. An analyte concentration is determined in a sample by a comparison value.

Despite the advantages involved in using mobile computing devices for the purpose of performing analytical measurements, several technical challenges remain. Specifically, reliability and accuracy of the measurements need to be enhanced and ensured. A major challenge is the presence and impact of varying environmental conditions, such as lighting conditions. Thus, measurement results may strongly be dependent on the environment to set up and/or background illumination and, thus, may vary from measurement to measurement, even under identical chemical or biochemical conditions. Furthermore, measurement results may depend on relative positioning of an illumination device and the camera of the mobile device which, due to a huge number of different mobile devices available on the market, may vary for different types or models of the mobile device. These technical challenges are even emphasized by the fact that, typically, when performing an analytical measurement by using optical test strips, at least two images need to be taken and analyzed, wherein one image shows at least part of a test field without having the sample applied thereto, whereas at least one second image is acquired having the sample applied thereto, wherein the application of the second image typically takes place after a certain time of waiting, until the color formation reaction has taken place. Since, in this case, at least two images need to be compared, wherein the test strip typically is handled and repositioned in between taking these two images the uncertainty of the measurement is additionally increased.

### Problem to be solved

It is therefore desirable to provide methods, computer programs and devices, which address the above-mentioned technical challenges of analytical measurements using mobile devices such as consumer-electronics mobile devices, specifically multipurpose mobile devices which are not dedicated to analytical measurements such as smart phones or tablet computers. Specifically, methods, computer programs and devices shall be proposed which ensure reliability and accuracy of the measurements.

### Summary

This problem is addressed by methods, computer programs, computer-readable storage media, mobile devices and kits for performing an analytical measurement with the features of the independent claims. Advantageous embodiments, which might be realized in an isolated fashion or in any arbitrary combinations are listed in the dependent claims.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, it shall be noted that the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, non-withstanding the fact that the respective feature or element may be present once or more than once.

Further, as used in the following, the terms "preferably", "more preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in such way with other optional or non-optional features of the invention.

In a first aspect, a method of performing an analytical measurement based on a color formation reaction in an optical test strip according to claim 1 is disclosed.

The term "analytical measurement" as used herein is a broad term and is to be given its ordinary and customary meaning to a person of ordinary skill in the art and is not to be limited to a special or customized meaning. The term specifically may refer, without limitation, to a quantitatively and/or qualitatively determination of at least one analyte in an arbitrary sample. The sample comprises a bodily fluid, such as blood, interstitial fluid, urine, saliva or other types of body fluids. The result of the analytical measurement, as an example, may be a concentration of the analyte and/or the presence or absence of the analyte to be determined. In particular, the analyte may be glucose. Specifically, as an example, the analytical measurement may be a blood glucose measurement, thus the result of the analytical measurement may be a blood glucose concentration. In particular, an analytical measurement result value may be determined by the analytical measurement. The term "analytical measurement result value" as used herein is a broad term and is to be given its ordinary and customary meaning to a person of ordinary skill in the art and is not to be limited to a special or customized meaning. The term specifically may refer, without limitation, to an arbitrary numerical indication of an analyte concentration in a sample.

The at least one analyte, as an example, may be or may comprise one or more specific chemical compounds and/or other parameters. As an example, one or more analytes may be determined which take part in metabolism, such as blood glucose. Additionally or alternatively, other types of analytes or parameters may be determined, e.g. a pH value. The at least one sample, specifically, may be or may comprise at least one bodily fluid, such as blood, interstitial fluid, urine, saliva or the like. Additionally or alternatively, however, other types of samples may be used, such as water.

The term "sample" as used herein is a broad term and is to be given its ordinary and customary meaning to a person of ordinary skill in the art and is not to be limited to a special or customized meaning. The term specifically may refer, without limitation, to an arbitrary amount of fluid for use in an analytical measurement. In particular, the sample may be a sample of bodily fluid and may be or may comprise at least 2 microliter (µl) of bodily fluid, in one embodiment at least 5 microliter (µl) of bodily fluid, such as of one or more of blood, interstitial fluid, urine, saliva and other body fluids. Specifically, the sample of bodily may comprise at least a minimum amount of bodily fluid necessary for performing an analytical measurement, specifically a minimum amount of bodily fluid for representatively determining the analyte concentration in the bodily fluid.

The analytical measurement, specifically, may be an analytical measurement including a change of at least one optical property of an optical test strip, which change may be measured or determined visually by using the camera. Specifically, the analytical measurement may be or may comprise a color formation reaction in the presence of the at least one analyte to be determined. The term "color formation reaction" as used herein is a broad term and is to be given its ordinary and customary meaning to a person of ordinary skill in the art and is not to be limited to a special or customized meaning. The term specifically may refer, without limitation, to a chemical, biological or physical reaction during which a color, specifically a reflectance, of at least one element involved in the reaction, changes with the progress of the reaction.

The term "optical test strip" as used herein is a broad term and is to be given its ordinary and customary meaning to a person of ordinary skill in the art and is not to be limited to a special or customized meaning. The term specifically may refer, without limitation, to an arbitrary element or device comprising at least one strip-shaped carrier, with the at least one test field applied thereto or integrated therein, the element being configured for performing a color-change detection reaction. The optical test strip may also be referred to as a test strip or a test element. The optical test strip may particularly have a test field containing at least one test chemical, such as at least one reagent element, for detecting at least one analyte. The optical test strip, as an example, may comprise at least one substrate, such as at least one carrier, with the at least one test field applied thereto or integrated therein. In particular, the optical test strip may further comprise at least one white area, such as a white field, specifically in a proximity to the test field, for example enclosing or surrounding the test field. The white area may be a separate field independently arranged on the substrate or carrier. However, additionally or alternatively, the substrate or carrier itself may be or may comprise the white area. These test strips are generally widely in use and available. One test strip may carry a single test field or a plurality of test fields having identical or different test chemicals comprised therein.

In step c) the sample of bodily fluid is applied to the test field of the optical test strip. As an example, at least one drop of sample, e.g. at least 2 to 5 µl of bodily fluid, may be applied to the test field. For example, the sample may be dropped and/or spread onto the test field. Various application techniques may be possible, such as, for example, applying the sample to the test field from a backside of the test field and capturing first and second images from the front side.

As further used herein, the term "test field" is a broad term and is to be given its ordinary and customary meaning to a person of ordinary skill in the art and is not to be limited to a special or customized meaning. The term specifically may refer, without limitation, to a coherent amount of the test chemical, such as to a field, e.g. a field of round, polygonal or rectangular shape, having one or more layers of material, with at least one layer of the test field having the test chemical comprised therein.

The term "mobile device" as used herein is a broad term and is to be given its ordinary and customary meaning to a person of ordinary skill in the art and is not to be limited to a special or customized meaning. The term specifically may refer, without limitation, to a mobile electronics device, more specifically to a mobile communication device such as a cell phone or smartphone. Additionally or alternatively, as will be outlined in further detail below, the mobile device may also refer to a tablet computer or another type of portable computer having at least one camera.

The term "camera" as used herein is a broad term and is to be given its ordinary and customary meaning to a person of ordinary skill in the art and is not to be limited to a special or customized meaning. The term specifically may refer, without limitation, to a device having at least one imaging element configured for recording or capturing spatially resolved one-dimensional, two-dimensional or even three-dimensional optical data or information. As an example, the camera may comprise at least one camera chip, such as at least one CCD chip and/or at least one CMOS chip configured for recording images. As used herein, without limitation, the term "image" specifically may relate to data recorded by using a camera, such as a plurality of electronic readings from the imaging device, such as the pixels of the camera chip.

The camera, besides the at least one camera chip or imaging chip, may comprise further elements, such as one or more optical elements, e.g. one or more lenses. As an example, the camera may be a fix-focus camera, having at least one lens which is fixedly adjusted with respect to the camera. Alternatively, however, the camera may also comprise one or more variable lenses which may be adjusted, automatically or manually. The invention specifically shall be applicable to cameras as usually used in mobile applications such as notebook computers, tablets or, specifically, cell phones such as smart phones. Thus, specifically, the camera may be part of a mobile device which, besides the at least one camera, comprises one or more data processing devices such as one or more data processors. Other cameras, however, are feasible.

The camera specifically may be a color camera. Thus, such as for each pixel, color information may be provided or generated, such as color values for three colors R, G, B. a larger number of color values is also feasible, such as four color values for each pixel, for example R, G, G, B. Color cameras are generally known to the skilled person. Thus, as an example, the camera chip may consist of a plurality of three or more different color sensors each, such as color recording pixels like one pixel for red (R), one pixel for green (G) and one pixel for blue (B). For each of the pixels, such as for R, G, B, values may be recorded by the pixels, such as digital values in the range of 0 to 255, depending on the intensity of the respective color. Instead of using color triples such as R, G, B, as an example, quadruples may be used, such as R, G, G, B. The color sensitivities of the pixels may be generated by color filters or by appropriate intrinsic sensitivities of the sensor elements used in the camera pixels. These techniques are generally known to the skilled person.

Steps b) and d) each comprise capturing at least one image by using the camera. The term "capturing at least one image" may refer to one or more of imaging, image recording, image acquisition, image capturing. The term "capturing at least one image" may comprise capturing a single image and/or a plurality of images such as a sequence of images. For example, the capturing of the image may comprise recording continuously a sequence of images such as a video or a movie. The capturing of the at least one image may be initiated by the user action or may automatically be initiated, e.g. once the presence of the at least one object within a field of view and/or within a predetermined sector of the field of view of the camera is automatically detected. These automatic image acquisition techniques are known e.g. in the field of automatic barcode readers, such as from automatic barcode reading apps. The capturing of the images may take place, as an example, by acquiring a stream or "live stream" of images with the camera, wherein one or more of the images, automatically or by user interaction such as pushing a button, are stored and used as the at least one first image or the at least one second image, respectively. The image acquisition may be supported by a processor of the mobile device, and the storing of the images may take place in a data storage device of the mobile device.

In each of steps b) and d), at least one image of at least a part of the test field is captured, by using the camera. These images are referred to as "the at least one first image" and "the at least one second image", wherein the terms "first" and "second" are used for the purpose of nomenclature, only, without ranking or numbering these images and without giving any preferences. The term "of at least a part of the test field" or "of at least part of the test field" both refer to the fact that at least one part of the at least one test field should be visible in each of the images, wherein, in the first and second images, different parts of the at least one test field may be visible. Besides the at least one part of the test field, in each case, further parts of the optical test strip may be visible, such as at least one part of a substrate of the test strip.

In step b), the at least one first image is captured without having a sample applied to the test field. This at least one first image typically is also referred to as the "blank image", and, in typical evaluation methods, the image is used for reference purposes, in order to take into account variations of the color or other optical properties of the test field which are not due to the sample or the analyte itself. The sample application in step c) may take place, as an example, directly or indirectly, e.g. via at least one capillary element. The at least one second image, captured after sample application, is typically also referred to as the "wet image", even though the sample may have dried when the image is actually captured. The second image typically is taken after having waited for at least a predetermined waiting time, such as after five seconds or more, in order to allow for the detection reaction to take place.

Thus, as an example, between performing steps c) and d) of the method, a minimum amount of waiting time may elapse. This minimum amount of waiting time specifically may be sufficient for a detection reaction to take place in the test strip. As an example, the minimum amount of waiting time may be at least 5 s.

In step e) at least one item of admissibility information is determined, wherein the item of admissibility information indicates admissibility only if the position of the mobile device for capturing the first image is substantially the same as the position of the mobile device for capturing the second image. The term "position" as used herein and as specifically used in the context of capturing the first and second images with the mobile device, is a broad term and is to be given its ordinary and customary meaning to a person of ordinary skill in the art and is not to be limited to a special or customized meaning. The term specifically may refer, without limitation, to at least one spatial information regarding the camera, e.g. the camera of the mobile device. The position of the mobile device may particularly refer to an absolute position in space. The position may be a position of the camera at the moment of capturing the image. The position may particularly refer to at least one of a spatial coordinate and/or a spatial orientation of the camera and/or mobile device. In particular, in case the position, such as the spatial information, of the mobile device, e.g. of the camera of the mobile device, is substantially the same for capturing the first and second images, the item of admissibility information indicates admissibility.

The term "admissibility" as used herein is a broad term and is to be given its ordinary and customary meaning to a person of ordinary skill in the art and is not to be limited to a special or customized meaning. The term specifically may refer, without limitation, to a characterization whether an element or device is permitted and/or denied for performing one or more predetermined functions. Thus, as an example, the admissibility may be qualified or quantified by using one or more position parameters of the device. These one or more position parameters may be compared with one or more conditions. As a simple example, one or more position parameters may be compared with one or more comparative values, reference values or standard values, wherein the comparison may result in a binary result such as "admissible" or "not admissible"/"inadmissible". As an example, the at least one comparative and/or reference value may comprise at least one threshold value, such as a maximum difference of the positions of the camera and/or mobile device when capturing the first image and the second image. The comparative values, reference values and/or standard values may be derived, as an example, from experiments or from boundary conditions determined e.g. by a precision to be achieved in the analytical measurement.

The term "item of admissibility information" as used herein is a broad term and is to be given its ordinary and customary meaning to a person of ordinary skill in the art and is not to be limited to a special or customized meaning. The term specifically may refer, without limitation, to an indication of information regarding the admissibility. In particular, the item of admissibility information may refer to an indication of the admissibility of determining an analytical measurement result value from the first image and the second image captured by using the camera of the mobile device and/or to an indication of the admissibility of the camera of the mobile device for capturing the second image. The item of admissibility information, as an example, may be Boolean or digital information, such as indicating "admissible" or "not admissible"/"inadmissible". Thus, as an example, in case the position of the mobile device, e.g. of the camera of the mobile device, when capturing the second image has changed more than a predetermined maximum tolerance from the position of the mobile device when capturing the first image, the capturing of the second image and/or the second image itself, specifically in case the image is already captured, may be determined as being inadmissible for the purpose of determining an analytical measurement result value.

The item of admissibility information specifically indicated admissibility in case the position of the mobile device is substantially the same for capturing the first and the second image. As used herein, the term "substantially the same", specifically as used in the context of the position of the mobile device, is a broad term and is to be given its ordinary and customary meaning to a person of ordinary skill in the art and is not to be limited to a special or customized meaning. The term specifically may refer, without limitation, to the fact that the first and second images are taken at positions which fulfill at least one predetermined or determinable similarity criterion. Thus, according to the claimed invention the positions of the mobile device when capturing the first and second images may be identical at least within a predetermined range of tolerance, such as a predetermined range of tolerance stored in at least one data storage device of the mobile device.

Specifically, the predetermined range of tolerance may be measurable by at least one sensor, for example by at least one acceleration sensor, of the mobile device. In particular, the predetermined range of tolerance may be measurable by at least one acceleration sensor of the mobile device, wherein the acceleration sensor may be configured for measuring acceleration of the mobile device in an arbitrary coordinate system, for example in one or more of a Cartesian coordinate system, a cylindrical coordinate system, a polar coordinate system and a spherical coordinate system. As an example, the predetermined range of tolerance may specifically be or may comprise a difference in position, such as a position difference r for example in a three-dimensional polar coordinate system and/or spherical coordinate system, 0 m ≤ r ≤ 0.05 m, specifically 0 m ≤ r ≤ 0.03 m, more specifically 0 m ≤ r ≤ 0.01 m.

As further indicated above, in step e) the at least one item of admissibility information is determined based on one or both of position sensor data and local position data

The position sensor data, as an example, may be data retrieved from the position sensor of the mobile device. The term "position sensor" as used herein is a broad term and is to be given its ordinary and customary meaning to a person of ordinary skill in the art and is not to be limited to a special or customized meaning. The term specifically may refer, without limitation, to an arbitrary element which is adapted to determine spatial information of the camera of the mobile device, e.g. adapted to detect a location and/or a change of location. The position sensor may be at least one position sensor of the mobile device and may thus at least partly be located within the mobile device. **In** particular, the position sensor of the mobile device may specifically be configured for detecting a location and/or a change of location of the mobile device. Specifically, the position sensor may be configured for generating position sensor data.

As used herein, the term "position sensor data" is a broad term and is to be given its ordinary and customary meaning to person of ordinary skill in the art and is not to be limited to a special or customized meaning. The term specifically may refer, without limitation, to an arbitrary form of information, such as a signal, generated by the position sensor, the information indicating a location and/or a change of location. As an example, the position sensor data generated by the position sensor may be or may comprise at least one electronic signal, such as at least one voltage and/or at least one current, according to the location and/or change of location. Thus, the position sensor data may be or may comprise at least one signal generated by the position sensor of the mobile device indicating the location of the mobile device quantitatively and/or qualitatively. As an example, the position sensor may be or may comprise one or more of a gyroscope, a motion sensor, an accelerometer, a Hall sensor, a barometer.

The term "local position data" as used herein is a broad term and is to be given its ordinary and customary meaning to a person of ordinary skill in the art and is not to be limited to a special or customized meaning. The term specifically may refer, without limitation, to a spatial information, such as to at least one item of spatial information, regarding one or more of the camera or the test field, wherein the at least one spatial information takes into account at least one environmental feature. Specifically, the local position data may be or may comprise spatial information referring to a position of at least one environmental feature in a field of view of the camera. The local position data may refer to the position of one or more of the camera or the test field when and/or during capturing the image, such as at the moment of capturing the image. As an example, the local position data may be or may comprise spatial information referring to at least one of a spatial coordinate and/or a spatial orientation, such as at least one spatial coordinate and/or at least one spatial orientation in at least one coordinate system defined by the at least one environmental feature. The local position data may, for example, be derived from an image, such as from one or both of the first image and the second image, captured by using the camera. The local position data in contrast to the position sensor data may be determined in relation to external elements, such as in relation to environmental features, and may be derived independent from the position sensor. For example, the local position data may be determined by image analysis comprising, for example, object recognition software and the like.

The term "environmental feature" as used herein is a broad term and is to be given its ordinary and customary meaning to a person of ordinary skill in the art and is not to be limited to a special or customized meaning. The term specifically may refer, without limitation, to any reference element and/or reference characteristic in a field of view of the camera. The environmental feature specifically may be suited for defining a location and/or a coordinate system in space and/or which may be used as a location marker in the field of view of the camera. The environmental feature specifically may be a feature which, between capturing the first and second images, has a fixed and/or unchanged position. The fixed and/or unchanged position of the environmental feature may particular refer to a fixed and/or unchanged absolute position in space. The environmental feature specifically may be a feature which, between capturing the first and second images, is likely not to change position. The environmental feature may be or may comprise an article or a part thereof, e.g. a table or a part of the table, such as a surface structure of the table. The at least one environmental feature specifically may comprise at least one of an article in the field of view of the camera or a structural feature of an article in the field of view of the camera. The environmental feature or the article may be tangible. The environmental feature according to the claimed invention is different from the test strip, or parts of the test strip, and from the mobile device having a camera or parts thereof.

For detecting the at least one environmental feature, the method may comprise detecting the at least one environmental feature in one or both of the first and second images. For this purpose, as an example, the method may make use of image recognition, such as software-based automatic image recognition and/or image recognition by machine learning processes.

The method may further comprise:
g) if the item of admissibility information indicates inadmissibility, performing one or both of:
- displaying an error message on the display of the mobile device; and
- aborting the method of performing an analytical measurement.

The mobile device specifically has at least one display. As used herein, the term "display", specifically used in the context of the mobile device, is a broad term and is to be given its ordinary and customary meaning to a person of ordinary skill in the art and is not to be limited to a special or customized meaning. The term specifically may refer, without limitation, to an illustrating user interface configured for representing information in a visual form. In particular, the term display may refer to the screen of a smartphone or portable tablet or laptop. Specifically, the display of the mobile device may have a flat and/or even surface. As an example, the display may be a liquid-crystal display (LCD), such as a flat-panel display, e.g. an electronically modulated optical device, using light-modulating properties of liquid crystals. Other types of displays may be possible, such as light-emitting diode (LED) displays or the like.

In step g), as an example, an error message may be displayed on the display of the mobile device, in case the item of admissibility information indicates inadmissibility. Thus, in case inadmissibility is indicated by the item of admissibility information, the display may show a note addressing a user and informing the user about an error, for example about the inadmissibility. Additionally or alternatively, in step g), if inadmissibility is indicated by the item of admissibility, the method of performing an analytical measurement may be aborted. Thus, in case the item of admissibility indicates inadmissibility, the method may be terminated.

Step e) of the method according to the claimed invention further comprises retrieving at least one first and at least one second item of position information of the mobile device. Specifically, step e) comprises retrieving the at least one first item of position information comprising information on the position of the mobile device when capturing the first image in step b) of the method. Further, step e) comprises retrieving at least one second item of position information comprising information on the position of the mobile device when capturing the second image in step d).

In particular, the first item of position information may be or may comprise the at least one position sensor data of the mobile device when capturing the first image in step b). Thus, as an example, the first item of position information may be retrieved from the position sensor of the mobile device.

The second item of position information specifically may be or may comprise the at least one position sensor data of the mobile device when capturing the second image in step d). Thus, as an example, the second item of position information may be retrieved from the position sensor of the mobile device.

As an example, the first item of position information may be or may comprise the at least one local position data, e.g. the local position data of the mobile device relative to the test field, when capturing the first image in step b). Thus, as an example, the first item of position information may be retrieved from the first image captured by using the mobile device.

In particular, the second item of position information specifically may be or may comprise the at least one local position data of the mobile device when capturing the second image in step d). Thus, as an example, the second item of position information may be retrieved from the second image captured by using the mobile device.

In particular, step e) may further comprise comparing the second item of position information with the first item of position information. Specifically, in step e), e.g. for the purpose of determining the at least one item of admissibility information, the first and second item of position information may be compared with each other.

According to the claimed invention, the item of admissibility information indicates admissibility in case the second item of position information is, at least within a predetermined range of tolerance, identical to the first item of position information. Specifically, the item of admissibility information may indicate admissibility in case the second item of position information is substantially the same as the first item of position information. Otherwise, such as in case the second item of position information is not identical, e.g. not within a predetermined range of tolerance, to the first item of position information, the item of admissibility information indicates inadmissibility.

The local position data may specifically comprise information on at least one of: a relative position between the camera and at least one environmental feature in a field of view of the camera; a relative position between the test field and at least one environmental feature in a field of view of the camera; a relative position between the camera and the test field in a coordinate system defined by at least one environmental feature in a field of view of the camera; a relative orientation between the camera and at least one environmental feature in a field of view of the camera; a relative orientation between the test field and at least one environmental feature in a field of view of the camera; a relative orientation between the camera and the test field in a coordinate system defined by at least one environmental feature in a field of view of the camera. Additionally, the local position data may comprise information on a relative position between the camera and the test field and/or on a relative orientation between the camera and the test field.

As used herein, the term "relative position" may specifically refer to a comparative location in space measurable by distance only. In particular, the relative position, in contrast to the relative orientation, may be measurable independent of a consideration of a rotation. Thus, the relative position between the camera and an arbitrary object may, for example, refer to a comparative location between a center of gravity of the camera, for example a center of gravity of the mobile phone comprising the camera, and a center of gravity of the arbitrary object. As an example, the relative position between the camera and the test field may refer to a comparative location between the camera and the test field irrespective of any rotation.

The term "relative orientation" as used herein may specifically refer to a comparative alignment in space measurable by rotation only. In particular, the relative orientation may be measurable independent of a consideration of a distance. Thus, the relative orientation between the camera and an arbitrary object may, for example, refer to a comparative rotation between a coordinate system located at the center of gravity of the camera, for example a coordinate system located at the center of gravity of the mobile device comprising the camera, and a coordinate system located at the center of gravity of the arbitrary object. In particular, as an example, the relative orientation between the camera and the test field may refer to a comparative rotational difference between a coordinate system of the camera and a coordinate system of the test field, specifically between coordinate systems located respectively at the center of gravity of the camera and the test field.

The method may further comprise a step of waiting for the mobile device to be at rest before performing step b) of capturing the first image. The term "at rest" as used herein, specifically used in context with the mobile device, is a broad term and is to be given its ordinary and customary meaning to a person of ordinary skill in the art and is not to be limited to a special or customized meaning. The term specifically may refer, without limitation, to a temporary state of unchanging location, such as to a temporary stillness regarding the position of an arbitrary object. In particular, the mobile device being at rest may refer to a temporary state of the mobile device, wherein the position of the mobile device remains unchanged, at least within a predetermined range of movement tolerance. As an example, the mobile device may be considered to be at rest in case the position of the mobile device remains within a range of ± 5 %, specifically within a range of ± 3 %, for at least 1 s, preferably for at least 3 s.

The camera and the at least one display of the mobile device may both be positioned on the same side of the mobile device. Specifically, the camera may be a front camera of the mobile device. The front camera and the display, specifically the front camera and the display of the mobile device, may both be positioned on a front of the mobile device, such as on a front side of the mobile device. In particular, the display and the front camera may be positioned on a same side, in particular on the front side, of the mobile device.

In steps b) and d), specifically when performing steps b) and d) of the method, the mobile device may be positioned in a fixed position by one or both of:
- using a holder for the mobile device; and
- placing the mobile device on a fixed surface.

The term "fixed position" as used herein is a broad term and is to be given its ordinary and customary meaning to a person of ordinary skill in the art and is not to be limited to a special or customized meaning. The term specifically may refer, without limitation, to a constant location in space. The position may be given by numerical values in an arbitrary coordinate system, such as by numerical values of three-dimensional coordinates and/or angles. The term "fixed" may refer to fixed numerical values in said coordinate system. The position may be defined by three-dimensional coordinates and/or orientation in space. In particular, the fixed position may refer to a constant and/or permanent position in space.

In particular, using the holder and/or placing the mobile device on the fixed surface, may ensure that the mobile device is at rest when capturing the first image in step b) and/or when capturing the second image in step d). Specifically, using the holder and/or placing the mobile device on the fixed surface, for example, may ensure the position of the mobile device to be essentially the same for capturing the first and second image.

The fixed surface may be a surface selected from the group consisting of: a level surface, such as a tabletop, a seating surface, a floor and a shelf board; an inclined or sloped surface; a flat surface; an irregular surface. Specifically, the fixed surface may be or may comprise any surface suitable for physically supporting the mobile device, for example, against gravity, e.g. against a gravitational force.

When capturing the at least one first image in step b), the test field of the optical test strip may be illuminated by using the display of the mobile device. As an example, the display of the mobile device may be suitable for emitting light, such as to illuminate the test field, when using the mobile device for capturing the at least one first image.

When capturing the at least one second image in step d), the test field of the optical test strip may be illuminated by using the display of the mobile device. As an example, the display of the mobile device may be suitable for emitting light, such as to illuminate the test field, when using the mobile device for capturing the at least one second image.

In particular, for illuminating the test field at least one area of the display of the mobile device may be illuminated. Thus, the at least one area of the display may be suitable for emitting at least one minimum amount of light for illuminating the test field when sing the mobile device for capturing at least one image, specifically the at least one first and/or second image. In particular, the at least one area of the display of the mobile device illuminated for illuminating the test field may, for example, be or may comprise at least 10 % of the display, specifically of a total area and/or complete surface area of the display. Preferably, the at least one area of the test field illuminated for illuminating the test field may be or may comprise at least 15 % of the display. More preferably, the at least one area of the test field illuminated for illuminating the test field may be or may comprise at least 20 % of the display.

The method may further comprise:
h) providing indications on where to locate the optical test strip for capturing the first and/or the second image by using the mobile device.

In particular, the indication on where to locate the optical test strip for capturing the first image may differ from the indication on where to locate the optical test strip for capturing the second image. Specifically, the indication on where to locate the optical test strip may be provided in step h) so that the first and second images are taken at substantially the same positions.

As an example, step h) may comprise indicating the location on where to capture the first image. Step h) may furthermore comprise indicating the location on where to capture the second image. Additionally or alternatively, step h) may comprise indicating the location on where to capture the second image based on the location the first image was captured.

The indication may specifically be provided by using the display of the mobile device. As an example, the indication specifically may comprise visual indication on the display of the mobile device. Specifically the indication on where to locate the optical test strip of step h) may comprise superposing a live image of the camera on the display of the mobile device with a visual guidance.

The visual guidance may specifically be selected from the group consisting of: an outline of the test strip to be targeted; a pointer indicating the direction into which the test strip is to be positioned; at least one word or phrase instructing the positioning of the test strip.

In particular, the visual guidance superposed in the live image of the camera at least partly may be or may comprise an augmented reality. The term "augmented reality" as used herein is a broad term and is to be given its ordinary and customary meaning to a person of ordinary skill in the art and is not to be limited to a special or customized meaning. The term specifically may refer, without limitation, to a method of overlaying, on a display, a current image, live image or image stream of a scene with one or more items of additional information, such as one or more visual indicators or the like. Thus, as an example, the indication on where to locate the optical test strip may be provided by providing, on the display, one or more arrows, frames or lines indicating a preferred positioning of the camera and/or the test strip. Additionally or alternatively, text may be displayed, indicating in which direction the camera and/or the test strip may have to be moved. Other visual guidance, e.g. other augmented reality, is possible.

Step d) of the method may comprise capturing a plurality of second images. Specifically, the method may comprise monitoring reaction kinetics by using the plurality of second images. As an example, the plurality of second images may be used for monitoring the reaction kinetics. In one embodiment the method may comprise determining a wetting induced change in the optical properties of the test field.

For example, the reaction kinetics may comprise at least one of a wetting-induced change and a detection reaction-induced change, such as a change in at least one optical property or in at least one item of optical information. In particular, the plurality of second images may be captured in order to determine if the at least one wetting-induced change occurred. In particular, a plurality of second images may be captured and the analytical measurement value may be determined based at least on one of the plurality of second images that was taken in a predefined time span (e.g., 3 to 8 seconds or 5 to 8 seconds) from one or more images being taken that are indicative of the start of the wetting-induced change. Thereby, measurement performance may be improved. Detecting the wetting-induced change based on the plurality of second images may serve as safeguard to exclude too short or overly long reaction times of the sample with the reagent system as too short or overly long reaction times may lead to wrong analytical measurement results. While the method may also comprises a step asking the user to confirm that a sample of bodily fluid was applied to the test field and receipt of this confirmation may be taken as start of the reaction, detecting the wetting-induced change automatically based on the plurality of second images is less bothersome for the user.

The method may comprise using at least one optical test strip, wherein the optical test strip may comprise at least one reagent element. The reagent element may specifically be configured so as to carry out at least one optically detectable detection reaction in the presence of the analyte.

Further, the method may comprise determining a time course of at least one optical measurement variable. The time course of the optical measurement variable may comprise a first time frame which comprises a sudden wetting-induced change (independent of the presence of the analyte) in the optical measurement variable. In particular, the time course of the optical measurement variable may further comprise a second time frame which may be subsequent to the first time frame. The second time frame may comprise a reaction kinetic (of the detection reaction of the reagent element in the presence of the analyte) used for determining the concentration of the analyte.

The test field of the optical test strip may be arranged on a detection side of the optical test strip. The optical test strip may further comprise a sample application side, wherein the sample of bodily fluid may be applied, e.g. by dropping and/or spreading the sample, to the test field from the sample application side. In particular, the sample application side may specifically be arranged opposite of the detection side, e.g. on a side facing an opposing direction than the detection side. Thus, the sample of bodily fluid may be applied to the test field from the sample application side, such as by dropping and/or spreading the sample onto the sample application side, e.g. onto a backside of the test field.

The method, in one or more embodiments disclosed, may be fully or partially computer-implemented. Thus, in a further aspect, a computer program is proposed comprising instructions which, when the program is executed by a mobile device having a camera, specifically by a processor of the mobile device, cause the mobile device to carry out the method as described herein, more specifically at least steps e) and f), and optionally steps b) and/or d), of the method. Further, also steps a) and c) of the method may, at least partially, be computer-implemented or at least computer-supported.

The computer program specifically may be designed as an application, e.g. as an App. The App, as an example, may be downloaded onto the mobile device from a download server.

As generally used herein, a "computer" may refer to a device having at least one processor and optionally further elements, such as one or more interfaces, one or more data storage devices, one or more user interfaces and the like. The term "processor" as used herein is a broad term and is to be given its ordinary and customary meaning to a person of ordinary skill in the art and is not to be limited to a special or customized meaning. The term specifically may refer, without limitation, to an arbitrary logic circuitry configured for performing basic operations of a computer or system, and/or, generally, to a device which is configured for performing calculations or logic operations. In particular, the processor may be configured for processing basic instructions that drive the computer or system. As an example, the processor may comprise at least one arithmetic logic unit (ALU), at least one floating-point unit (FPU), such as a math coprocessor or a numeric coprocessor, a plurality of registers, specifically registers configured for supplying operands to the ALU and storing results of operations, and a memory, such as an L1 and L2 cache memory. In particular, the processor may be a multi-core processor. Specifically, the processor may be or may comprise a central processing unit (CPU). Additionally or alternatively, the processor may be or may comprise a microprocessor, thus specifically the processor's elements may be contained in one single integrated circuitry (IC) chip. Additionally or alternatively, the processor may be or may comprise one or more application-specific integrated circuits (ASICs) and/or one or more field-programmable gate arrays (FPGAs) or the like.

The computer program may further comprise instructions that, when the program is executed by the mobile device, further prompt a used to perform one or both of steps a) and c) or to confirm having performed one or both of steps a) and c).

In a further aspect, a computer-readable storage medium is disclosed, specifically a non-transitory storage medium, comprising instructions which, when executed by a mobile device having a camera, specifically by a processor of the mobile device, cause the mobile device to carry out the method according to the present invention, such as according to any one of the embodiments disclosed above and/or according to any one of the embodiments disclosed in further detail below. Specifically, at least steps e) and f) of the method may be performed, wherein also one or more of steps a), b) c) and d) may at least partially be computer-implemented or at least computer-supported. Thus, the computer-readable storage medium may further comprise instructions which, when executed by the mobile device, further prompt a user to perform one or more of steps a), b) c) and d) or to confirm having performed one or more of steps a), b) c) and d).

As used herein, the terms "computer-readable data carrier" and "computer-readable storage medium" specifically may refer to non-transitory data storage means, such as a hardware storage medium having stored thereon computer-executable instructions. The computer-readable data carrier or storage medium specifically may be or may comprise a storage medium such as a random-access memory (RAM) and/or a read-only memory (ROM).

The computer program may also be embodied as a computer program product. As used herein, a computer program product may refer to the program as a tradable product. The product may generally exist in an arbitrary format, such as in a paper format, or on a computer-readable data carrier and/or on a computer-readable storage medium. Specifically, the computer program product may be distributed over a data network.

**In** a further aspect, a mobile device for performing an analytical measurement is disclosed. For definitions and options of the mobile device, reference may be made to the description of the method given above or as further outlined below. The mobile device comprises at least one camera, at least one display and at least one position sensor and may comprise one or more processors. The mobile device is configured for performing at least steps e) and f), and optionally steps b) and/or d), of the method of performing an analytical measurement according to the present invention, such as according to any one of the embodiments disclosed above and/or according to any one of the embodiments described in further detail below. Thus, the processor of the mobile device may be software-configured for performing and/or controlling the execution of the method, at least one of steps e) and f) of the method of performing an analytical measurement, wherein also steps a), b) c) and/or d) may at least partially be controlled and/or supported by the processor.

As outlined above, the mobile device may comprise at least one processor being programmed for controlling at least one of steps e) and f), and optionally steps b) and/or d), of the method of performing an analytical measurement. For definitions and options regarding the design of the processor, reference may be made to the description given above.

In a further aspect, a kit for performing an analytical measurement is disclosed. The kit comprises:
- at least one mobile device as described herein above or as described in further detail below; and
- at least one optical test strip having at least one test field.

The term "kit" as used herein is a broad term and is to be given its ordinary and customary meaning to a person of ordinary skill in the art and is not to be limited to a special or customized meaning. The term specifically may refer, without limitation, to an assembly of a plurality of components, wherein the components each may function and may be handled independently from each other, wherein the components of the kit may interact to perform a common function.

The invention in any one of the aspects described herein may provide for a large number of advantages over known methods and devices of this kind. Thus, specifically, the invention may address the technical challenges referred to above. As outlined above, smartphone-based methods typically require capturing at least two images, wherein at least one image is taken before sample application and at least one thereafter, which may be referred to as the "wet" or final image.

The present invention may allow for an improved measurement security of analytical measurements. Thus, the measurement security may be improved by monitoring reaction kinetics when performing the analytical measurements. In particular, the use of a front camera of the mobile device, e.g. smartphone, may allow for an exact determination of the time of application of the sample onto the test field of the optical test strip. The time of application may specifically be or may comprise a beginning of a reaction, such as a chemical reaction, e.g. a color-change reaction of the test field, and may thus be relevant for measurement performance. In particular, the measurement performance may be improved by allowing for an exact determination of the time of application.

Further, the present invention may improve measurement performance by monitoring reaction kinetics. Specifically, the present invention, by monitoring and/or detecting wetting-induced change, may provide safeguarding against overly long and/or too short measuring times.

Specifically, measurement accuracy may be improved by allowing for the same or at least similar positioning of the test strip during capturing the first or blank image and the at least one second or final image acquisition after sample application. **In** particular, the measurement accuracy may be improved by allowing the mobile device, such as the smartphone, to have the same or at least similar position when capturing the first and second images, for example, by placing the smartphone on a fixed surface, e.g. on a table. Thus, specifically when using a front camera of the mobile device, e.g. the front camera of the smartphone, the mobile device may act as a fixed point for a user when positioning the optical test strip allowing for the same or at least similar positioning when capturing the images. In particular, the invention may greatly improve measurement accuracy by allowing the position and/or location of the smartphone to be checked and/or verified by at least one position sensor of the mobile device.

Generally, the invention may greatly improve measurement performance of analytical measurements. Thus, the measurement performance of smartphone-based optical analysis of test strips may typically strongly depend on the conditions under which the images before and after sample application are taken. Ideally, the conditions are the same for both images. For positioning, at least one position sensor of the mobile device and/or image recognition techniques may be used to determine the conditions, in order to improve measurement performance.

### Short description of the Figures

Further optional features and embodiments will be disclosed in more detail in the subsequent description of embodiments, preferably in conjunction with the dependent claims. Therein, the respective optional features may be realized in an isolated fashion as well as in any arbitrary feasible combination, as the skilled person will realize. The scope of the invention is not restricted by the preferred embodiments. The embodiments are schematically depicted in the Figures. Therein, identical reference numbers in these Figures refer to identical or functionally comparable elements.

In the Figures:
- Figure 1: shows an embodiment of a kit and a mobile device for performing an analytical measurement in a perspective view;
- Figure 2: shows an embodiment of a mobile device for performing an analytical measurement in a front view;
- Figures 3 to 5: show flowcharts of different embodiments of a method of performing an analytical measurement;
- Figure 6: exemplarily show a diagram of measured reaction kinetics; and
- Figure 7: shows comparative blood glucose measurements.

### Detailed description of the embodiments

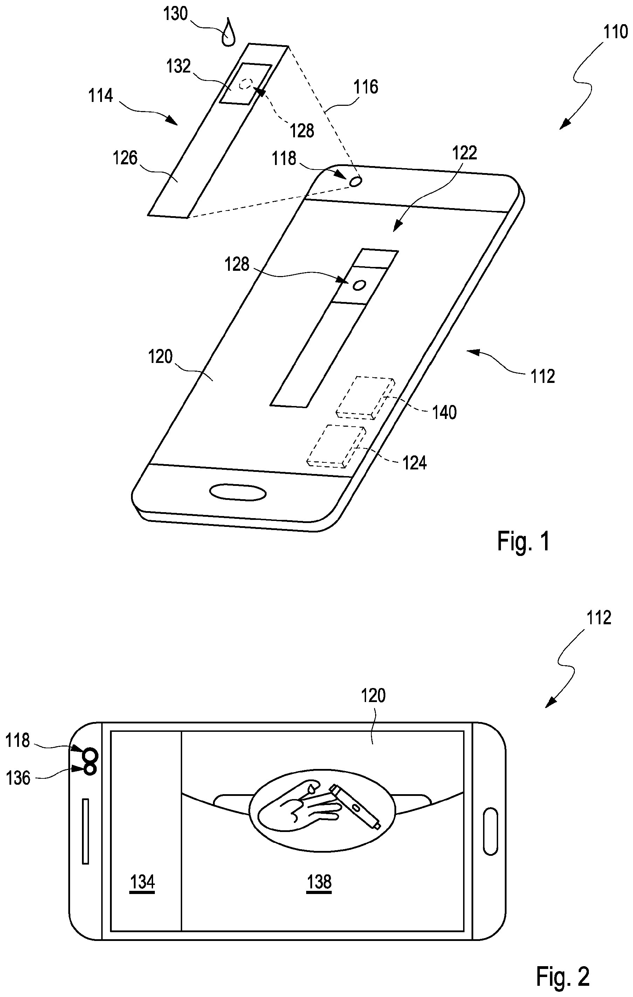

In Figure 1, an exemplary embodiment of a kit 110 for performing an analytical measurement is shown in a perspective view. The kit 110 comprises a mobile device 112, such as for example a smart phone, and further at least one optical test strip 114. In the illustrated set-up, the optical test strip 114 is placed in a field of view 116 of a camera 118 of the mobile device 112.

The mobile device 112, besides the at least one camera 118, comprises at least one display 120, wherein the display 120 may be configured for displaying a live image 122 taken by the camera 118 and/or for displaying information to a user. The mobile device 112 further comprises at least one position sensor 124, such as, for example, a position sensor 124 configured for detecting one or both of a position, e.g. a location, of the mobile device 112 and a change in the position, e.g. the location, of the mobile device 112.

The optical test strip 114 may comprise at least one substrate 126, such as a flexible, strip shaped substrate. The optical test strip 114 further comprises at least one test field 128 applied to the substrate, the test field 128 comprising at least one test chemical for performing a detection reaction with at least one analyte comprised by a sample 130, specifically by a sample 130 of bodily fluid. The sample may directly or indirectly be applied to the test field 128, such as by applying a droplet of the bodily fluid to the test field 128 and/or, as exemplarily illustrated in Figure 1, to a spreading aid 132 from which the sample 130 is conducted to the test field 128.

The display 120 of the mobile device 112, as exemplarily illustrated in Figure 2, may for example comprise a first area 134, which may be illuminated for illuminating the test field 128 of the optical test strip 114. Additionally or alternatively, the mobile device 112 may comprise at least one illumination source 136, such as an LED or the like, for illuminating the test field 128. Further, the display 120 may comprise a second area 138 for displaying information to the user.

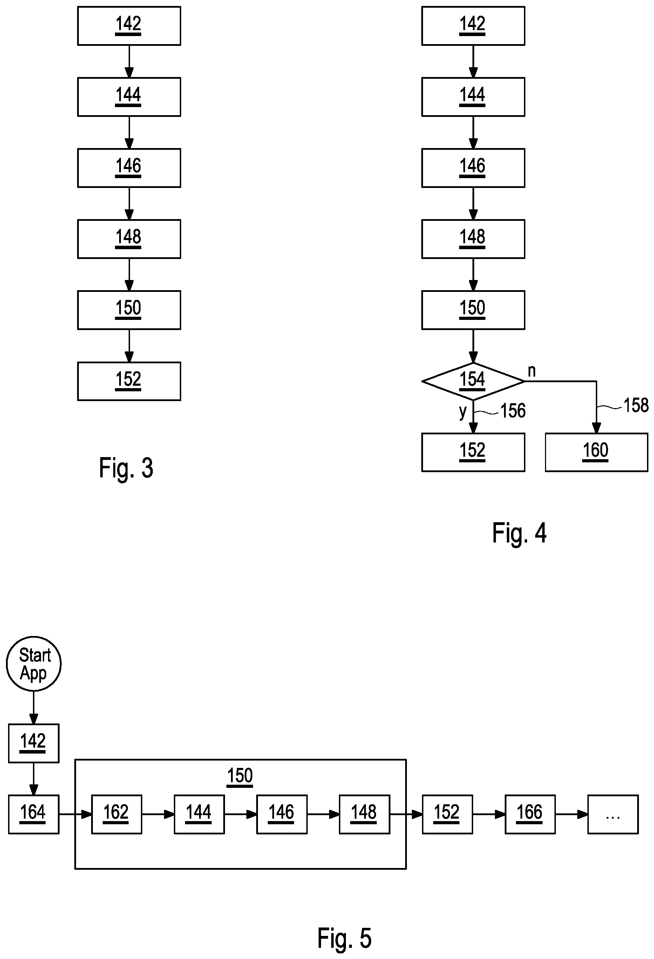

The mobile device 112 is configured, for example by appropriate programming of a processor 140 of the mobile device 112, for performing at least steps e) and f) of a method of performing an analytical measurement. The method will be described with reference to exemplary embodiments shown in flowcharts illustrated in Figures 3, 4 and 5.

The method of performing an analytical measurement based on a color formation reaction in an optical test strip 114 by using a mobile device 112 having a camera 118, at least one display 120 and a position sensor 124 comprises the following steps, which may specifically be performed in the given order. Still, a different order may also be possible. It may be possible to perform two or more of the method steps fully or partially simultaneously. It may further be possible to perform one, more than one or even all of the method steps once or repeatedly. The method may comprise additional method steps which are not listed. The method steps of the method are the following:
a) (denoted with reference number 142) providing a dry optical test strip 114 having a test field 128;
b) (denoted with reference number 144) capturing at least one first image of at least part of the test field 128 of the dry optical test strip 114 without having a sample 130 applied thereto by using the camera 118;
c) (denoted with reference number 146) applying a sample 130 of bodily fluid to the test field 128 of the optical test strip 114;
d) (denoted with reference number 148) capturing at least one second image of at least part of the test field 128 of the optical test strip 114 having the sample 130 applied thereto by using the camera 118;
e) (denoted with reference number 150) determining at least one item of admissibility information, wherein the item of admissibility information indicates admissibility in case the position of the mobile device 112 is substantially the same for capturing the first and the second image, wherein the item of admissibility information is determined based on one or both of position sensor data and local position data; and
f) (denoted with reference number 152) if the item of admissibility information indicates admissibility, determining an analytical measurement result value by using the first and the second image of the test field 128 of the optical test strip 114.

Further, as exemplarily illustrated in Figure 4, the method may comprise a branching point 154. The branching point 154 may indicate a condition query, such as deciding between a first branch 156 and a second branch 158. For example, the condition query may make use of the item of admissibility information. The item of admissibility information may comprise Boolean information, such as "admissible" ("y") or "inadmissible" ("n"). As an example, the first branch 156 indicates admissibility of determining an analytical measurement result value from the first image and the second image captured by using the camera 118 of the mobile device 112. Thus, the first branch 156 leads to step f), wherein the analytical measurement result value is determined by using the first and the second image of the test field 128 of the optical test strip 114.

The second branch 158 may indicate inadmissibility and, thus, may lead to step g) (denoted with reference number 160) if the item of admissibility information indicates inadmissibility, performing one or both of:displaying an error message on the display 120 of the mobile device 112; and aborting the method of performing an analytical measurement.

As illustrated in Figure 5, step e) 150 may, for example, be performed in parallel to other method steps, such as steps b) 144, c) 146 and d) 148, before determining the analytical measurement result value in step f) 152. Additionally, the method may comprise further steps, such as indicating a user to position the mobile device 112 in a fixed position (denoted with reference number 162), for example as by indicating to place a phone and/or smart phone, on a fixed surface, e.g. on a table. Specifically, performance of step e) may, for example, start with the mobile device, e.g. the smartphone, being placed on any flat support, for example on a table. Thus, the position sensor 124 of the mobile device 112, e.g. a smartphone sensor, may start monitoring movements of the mobile device, e.g. of the smartphone. Further the method may comprise a step (denoted with reference number 164) of requesting an analytical measurement, such as a blood glucose measurement, and a step (denoted with reference number 166) of displaying a result of the measurement. As an example, the result of the measurement displayed may be a range indication, indicating a range within which the analytical measurement has been detected. Additionally or alternatively, the result of the measurement displayed may be the analytical measurement result value. In particular, for example, the result of the measurement may be displayed on the display 120 of the mobile device 112. Further steps, such as informing a user that the phone must be on rest before a measurement sequence starts, though not illustrated in the Figures, may be possible.

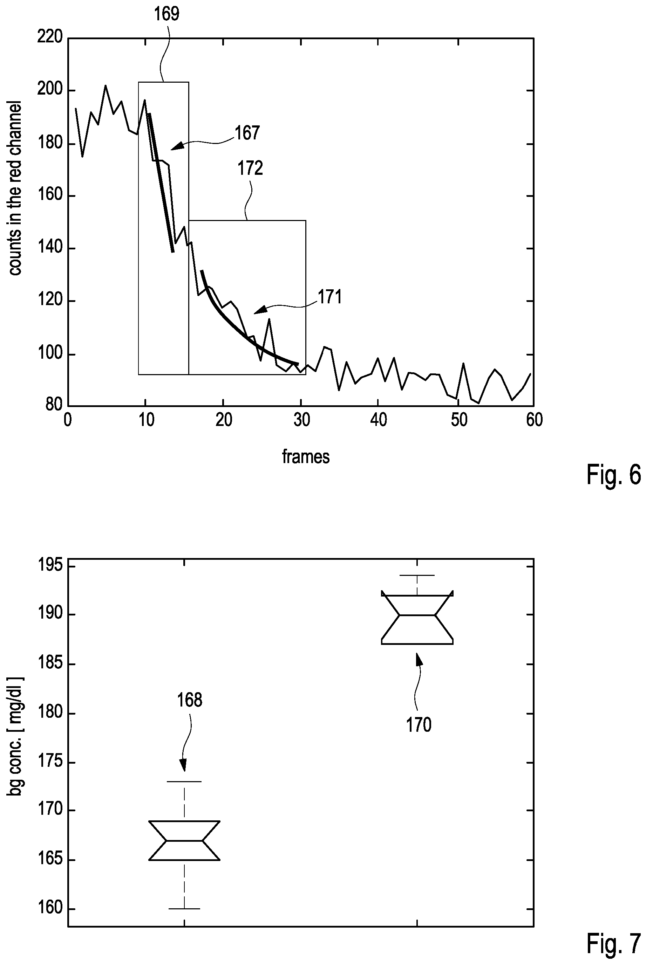

In Figure 6, an exemplary diagram of reaction kinetics is illustrated. For this experiment, the mobile phone 112 was kept in a fixed position while the test strip 114 was kept in a freehand manner in the front camera's field of view upon application of a sample to the test field. The x-axis in Fig. 6 shows the consecutive frames (measurement data points) taken; the y-axis shows the measured counts in the red channel. The number of measurement data points taken during the reaction is taking place may depend on the user handling, such as on the handling of the optical test strip 114 and/or the mobile device 112 by the user, in case where image capturing is triggered automatically. As an example, the automatically triggered image capturing may be or may comprise capturing a quantity of N images per second, wherein 1 ≤ N ≤ 15, specifically 3 ≤ N ≤ 12, more specifically 5 ≤ N ≤ 10.

Despite some noise in the signal being visible due to the freehand positioning of the test strip in the camera's field of view, the wetting induced drop in intensity can be clearly seen in the beginning. In particular, in the diagram illustrated in Figure 6, the wetting induced change, e.g. a wetting drop 167, may be or may comprise a change of more than 25 % in the measured counts in the red channel as illustrated on the y-axis. As an example, in Figure 6, the wetting induced change, such as the wetting drop 167, may be visible in a first time frame 169, e.g. from frames 10 to 16, wherein the reaction kinetic used for determining the concentration of the analyte 171 may be visible in a second time frame 172, e.g. from frames 16 to 30. Between frames 0 and 10 the measured counts in the red channel may vary less than 25 %, specifically less than 15 %. In particular, monitoring the wetting-induced change based on the plurality of second images may serve as safeguard to exclude too short or overly long reaction times of the sample with the reagent system. Furthermore, monitoring the wetting-induced change can also be used to determine the starting point of the chemical reaction and thus measure reaction times. The reaction time can then be considered in the determination of the analyte concentration.

In Figure 7, measurement results are shown which demonstrate the effect of controlling the local positions for capturing the blank image and the final image. For these experiments, blood glucose measurements were performed using an optical test strip 114 and a sample 130. Two different setups were used: In a first setup, denoted by reference number 168, for the blank images or first images and the final images or second images were taken at identical local positions. Specifically, in the first setup 168, the camera 118 was positioned in an identical location for the first and second images and the optical test strip 114 was positioned in an identical location for the first and second images. In a second setup, denoted by reference number 170, the blank images or first images were taken at a common first local position, and the final images or second images were taken at a common second local position, wherein the second local position differed from the first local position. Specifically, in the second setup 170, the position of the camera 118 was changed between the taking of the first and second images and the position of the optical test strip 114 was also changed between the taking of the first and second images. In each setup, 10 measurements were performed, wherein for the blank images a fresh optical test strip 114 was used (no sample applied), whereas for the final images an optical test strip 114 was used 3 days after sample application for demonstration purposes (the test field of this strip had constant optical properties different from a fresh optical test strip).

On the horizontal axis, the two different setups 168, 170 are shown in Figure 7. On the vertical axis, the determined analytical measurement result is shown, in this case a blood glucose concentration c in mg/dl. The results are shown as box plots for both setups 168, 170. As can be seen, a significant difference occurs between the correct or controlled setup 168 and the uncontrolled setup 170. The difference is supposed to be mainly due to differing illumination conditions in the first and second local positions. The difference clearly shows the benefit of the present invention, since taking the first and second images at similar local positions can provide for increased measurement performance, e.g., in terms of reproducibility and/or accuracy.

### List of reference numbers

- 110: Kit
- 112: mobile device
- 114: optical test strip
- 116: field of view of the camera
- 118: Camera
- 120: Display
- 122: live image taken by the camera
- 124: position sensor
- 126: Substrate
- 128: test field
- 130: Sample
- 132: spreading aid
- 134: first area
- 136: illumination source
- 138: second area
- 140: Processor
- 142: step a)
- 144: step b)
- 146: step c)
- 148: step d)
- 150: step e)
- 152: step f)
- 154: branching point
- 156: first branch
- 158: second branch
- 160: step g)
- 162: indicating a user to position the mobile device in a fixed position
- 164: requesting an analytical measurement
- 166: displaying the analytical measurement result value
- 167: wetting drop
- 168: first setup: blank image and final image taken at identical local positions
- 169: first time frame
- 170: second setup: blank image and final image taken at different local positions
- 171: reaction kinetic used for determining the concentration of the analyte
- 172: second time frame

## Claims

1. A method of performing an analytical measurement based on a color formation reaction in an optical test strip (114) by using a mobile device (112) having a camera (118), at least one display (120) and a position sensor (124), the method comprising:
a) providing a dry optical test strip (114) having a test field (128);
b) capturing at least one first image of at least part of the test field (128) of the dry optical test strip (114) without having a sample (130) applied thereto by using the camera (118);
c) applying a sample (130) of bodily fluid to the test field (128) of the optical test strip (114);
d) capturing at least one second image of at least part of the test field (128) of the optical test strip (114) having the sample (130) applied thereto by using the camera (118);
e) determining at least one item of admissibility information, wherein the item of admissibility information indicates admissibility in case the position of the mobile device (112) is substantially the same for capturing the first and the second image, wherein the item of admissibility information is determined based on one or both of position sensor data and local position data, wherein the local position data is or comprises spatial information referring to a position of at least one environmental feature in a field of view of the camera, wherein the environmental feature is different from the test strip, or parts of the test strip and from the mobile device or parts thereof; and
f) if the item of admissibility information indicates admissibility, determining an analytical measurement result value by using the first and the second image of the test field (128) of the optical test strip (114)
wherein step e) comprises retrieving at least one first item of position information comprising information on the position of the mobile device when capturing the first image in step b) and retrieving at least one second item of position information comprising information on the position of the mobile device when capturing the second image in step d),
wherein step e) further comprise comparing the first and second item of position information with each other, and wherein the item of admissibility information indicates admissibility in case the second item of position information is, at least within a predetermined range of tolerance, identical to the first item of position information.

2. The method according to the preceding claim, wherein the method further comprises
g) if the item of admissibility information indicates inadmissibility, performing one or both of:
- displaying an error message on the display (120) of the mobile device (112); and
- aborting the method of performing an analytical measurement.

3. The method according to any one of the preceding claims, wherein the camera (118) is a front camera of the mobile device (112), wherein the front camera and the at least one display (120) of the mobile device (112) are both positioned on a front of the mobile device (112).

4. The method according to any one of the preceding claims, wherein in steps b) and d) the mobile device (112) is positioned in a fixed position by one or both of:
- using a holder for the mobile device (112); and
- placing the mobile device (112) on a fixed surface.

5. The method according to the preceding claim, wherein the fixed surface is a surface selected from the group consisting of: a level surface, such as a tabletop, a seating surface, a floor and a shelf board; an inclined or sloped surface; a flat surface; an irregular surface.

6. The method according to any one of the preceding claims, wherein when capturing the at least one first image in step b), the test field (128) of the optical test strip (114) is illuminated by using the display (120) of the mobile device (112), wherein when capturing the at least one second image in step d), the test field (128) of the optical test strip (114) is illuminated by using the display (120) of the mobile device (112).

7. The method according to any one of the preceding claims, wherein the method further comprises:
h) providing indications on where to locate the optical test strip (114) for capturing the first and/or the second image by using the mobile device (112).

8. The method according to the preceding claim, wherein the indication is provided by using the display (120) of the mobile device (112), wherein the indication on where to locate the optical test strip (114) of step h) comprises superposing a live image of the camera (118) on the display (120) of the mobile device (112) with a visual guidance.

9. The method according to any one of the preceding claims, wherein step d) comprises capturing a plurality of second images, wherein the method comprises monitoring reaction kinetics by using the plurality of second images.

10. A computer program comprising instructions which, when the program is executed by a mobile device (112) having a camera (118) cause the mobile device (112) to carry out at least steps e) and f), and optionally steps b) and d), of the method according to any one of the preceding claims.

11. A computer-readable storage medium, specifically a non-transitory storage medium, comprising instructions which, when executed by a mobile device (112) having a camera (118) cause the mobile device (112) to carry out at least steps e) and f), and optionally steps b) and/or d), of the method according to any one of the preceding method claims.

12. A mobile device (112) for performing an analytical measurement, the mobile device (112) having at least one camera (118), at least one display (120) and a position sensor (124), the mobile device (112) being configured for performing at least steps e) and f), and optionally steps b) and/or d), of the method of performing an analytical measurement according to any one of the preceding claims referring to a method of performing an analytical measurement.

13. A kit (110) for performing an analytical measurement, the kit (110) comprising:
- at least one mobile device (112) according to any one of the preceding claims referring to a mobile device (112); and
- at least one optical test strip (114) having at least one test field (128).

## Patentansprüche

1. Verfahren zur Durchführung einer analytischen Messung basierend auf einer Farbbildungsreaktion in einem optischen Teststreifen (114) unter Verwendung einer mobilen Vorrichtung (112) mit einer Kamera (118), mindestens einer Anzeige (120) und einem Positionssensor (124), wobei das Verfahren Folgendes umfasst:
a) Bereitstellen eines trockenen optischen Teststreifens (114) mit einem Testfeld (128);
b) Aufnehmen mindestens eines ersten Bildes von mindestens einem Teil des Testfelds (128) des trockenen optischen Teststreifens (114) ohne eine darauf aufgegebene Probe (130) unter Verwendung der Kamera (118);
c) Aufgeben einer Probe (130) von Körperflüssigkeit auf das Testfeld (128) des optischen Teststreifens (114);
d) Aufnehmen mindestens eines zweiten Bildes von mindestens einem Teil des Testfelds (128) des optischen Teststreifens (114) mit der darauf aufgegebenen Probe (130) unter Verwendung der Kamera (118);
e) Bestimmen mindestens eines Elements von Zulässigkeitsinformationen, wobei das Element von Zulässigkeitsinformationen Zulässigkeit angibt, falls die Position der mobilen Vorrichtung (112) zum Aufnehmen des ersten und des zweiten Bildes im Wesentlichen die gleiche ist, wobei das Element von Zulässigkeitsinformationen basierend auf einem oder beiden von Positionssensordaten und lokalen Positionsdaten bestimmt wird, wobei die lokalen Positionsdaten räumliche Informationen sind oder umfassen, die sich auf eine Position mindestens eines Umgebungsmerkmals in einem Sichtfeld der Kamera beziehen, wobei sich das Umgebungsmerkmal von dem Teststreifen oder Teilen des Teststreifens und von der mobilen Vorrichtung oder Teilen davon unterscheidet; und
f) wenn das Element von Zulässigkeitsinformationen Zulässigkeit angibt, Bestimmen eines analytischen Messergebniswerts unter Verwendung des ersten und des zweiten Bildes des Testfelds (128) des optischen Teststreifens (114),
wobei Schritt e) das Abrufen mindestens eines ersten Elements von Positionsinformationen, die Informationen über die Position der mobilen Vorrichtung umfassen, wenn das erste Bild in Schritt b) aufgenommen wird, und das Abrufen mindestens eines zweiten Elements von Positionsinformationen, die Informationen über die Position der mobilen Vorrichtung umfassen, wenn das zweite Bild in Schritt d) aufgenommen wird, umfasst,
wobei Schritt e) ferner das Vergleichen des ersten und des zweiten Elements von Positionsinformationen miteinander umfasst und wobei das Element von Zulässigkeitsinformationen Zulässigkeit angibt, falls das zweite Element von Positionsinformationen mindestens innerhalb eines vorbestimmten Toleranzbereichs mit dem ersten Element von Positionsinformationen identisch ist.

2. Verfahren nach dem vorhergehenden Anspruch, wobei das Verfahren ferner Folgendes umfasst:
g) wenn das Element von Zulässigkeitsinformationen Nichtzulässigkeit angibt, Durchführen eines oder beides von Folgendem:
- Anzeigen einer Fehlermeldung auf der Anzeige (120) der mobilen Vorrichtung (112); und
- Abbrechen des Verfahrens zur Durchführung einer analytischen Messung.

3. Verfahren nach einem der vorhergehenden Ansprüche, wobei die Kamera (118) eine vordere Kamera der mobilen Vorrichtung (112) ist, wobei die vordere Kamera und die mindestens eine Anzeige (120) der mobilen Vorrichtung (112) beide auf einer Vorderseite der mobilen Vorrichtung (112) positioniert sind.

4. Verfahren nach einem der vorhergehenden Ansprüche, wobei in den Schritten b) und d) die mobile Vorrichtung (112) durch eines oder beides von Folgendem in einer festen Position positioniert wird:
- Verwenden eines Halters für die mobile Vorrichtung (112); und
- Platzieren der mobilen Vorrichtung (112) auf einer festen Fläche.

5. Verfahren nach dem vorhergehenden Anspruch, wobei die feste Fläche eine Fläche ist, die aus der Gruppe ausgewählt ist, die aus Folgendem besteht: einer ebenen Fläche, wie etwa einer Tischplatte, einer Sitzfläche, einem Boden und einem Regal; einer geneigten oder schrägen Fläche; einer glatten Fläche; einer unregelmäßigen Fläche.

6. Verfahren nach einem der vorhergehenden Ansprüche, wobei das Testfeld (128) des optischen Teststreifens (114) unter Verwendung der Anzeige (120) der mobilen Vorrichtung (112) beleuchtet wird, wenn das mindestens eine erste Bild in Schritt b) aufgenommen wird, wobei das Testfeld (128) des optischen Teststreifens (114) unter Verwendung der Anzeige (120) der mobilen Vorrichtung (112) beleuchtet wird, wenn das mindestens eine zweite Bild in Schritt d) aufgenommen wird.

7. Verfahren nach einem der vorhergehenden Ansprüche, wobei das Verfahren ferner Folgendes umfasst:
h) Bereitstellen von Angaben darüber, wo der optische Teststreifen (114) zum Aufnehmen des ersten und/oder des zweiten Bildes unter Verwendung der mobilen Vorrichtung (112) zu platzieren ist.

8. Verfahren nach dem vorhergehenden Anspruch, wobei die Angabe unter Verwendung der Anzeige (120) der mobilen Vorrichtung (112) bereitgestellt wird, wobei die Angabe darüber, wo der optische Teststreifen (114) von Schritt h) zu platzieren ist, das Überlagern eines Live-Bildes der Kamera (118) auf der Anzeige (120) der mobilen Vorrichtung (112) mit einer visuellen Führung umfasst.

9. Verfahren nach einem der vorhergehenden Ansprüche, wobei Schritt d) das Aufnehmen einer Vielzahl von zweiten Bildern umfasst, wobei das Verfahren das Überwachen der Reaktionskinetik unter Verwendung der Vielzahl von zweiten Bildern umfasst.

10. Computerprogramm, das Anweisungen umfasst, die, wenn das Programm von einer mobilen Vorrichtung (112) mit einer Kamera (118) ausgeführt wird, die mobile Vorrichtung (112) veranlassen, mindestens die Schritte e) und f) und optional die Schritte b) und d) des Verfahrens nach einem der vorhergehenden Ansprüche auszuführen.

11. Computerlesbares Speichermedium, insbesondere ein nichtflüchtiges Speichermedium, das Anweisungen umfasst, die, wenn sie von einer mobilen Vorrichtung (112) mit einer Kamera (118) ausgeführt werden, die mobile Vorrichtung (112) veranlassen, mindestens die Schritte e) und f) und optional die Schritte b) und/oder d) des Verfahrens nach einem der vorhergehenden Verfahrensansprüche auszuführen.

12. Mobile Vorrichtung (112) zur Durchführung einer analytischen Messung, wobei die mobile Vorrichtung (112) mindestens eine Kamera (118), mindestens eine Anzeige (120) und einen Positionssensor (124) aufweist, wobei die mobile Vorrichtung (112) zur Durchführung mindestens der Schritte e) und f) und optional der Schritte b) und/oder d) des Verfahrens zur Durchführung einer analytischen Messung nach einem der vorhergehenden Ansprüche, die sich auf ein Verfahren zur Durchführung einer analytischen Messung beziehen, konfiguriert ist.

13. Kit (110) zur Durchführung einer analytischen Messung, wobei der Kit (110) Folgendes umfasst:
- mindestens eine mobile Vorrichtung (112) nach einem der vorhergehenden Ansprüche, die sich auf eine mobile Vorrichtung (112) beziehen; und
- mindestens einen optischen Teststreifen (114) mit mindestens einem Testfeld (128).

## Revendications

1. Procédé de réalisation d'une mesure analytique basée sur une réaction de formation de couleur dans une bandelette de test optique (114) en utilisant un dispositif mobile (112) ayant une caméra (118), au moins un écran (120) et un capteur de position (124), le procédé comprenant :
a) la fourniture d'une bandelette de test optique (114) sèche ayant un champ de test (128) ;
b) la capture d'au moins une première image d'au moins une partie du champ de test (128) de la bandelette de test optique (114) sèche sans l'application d'un échantillon (130) sur celui-ci en utilisant la caméra (118) ;
c) l'application d'un échantillon (130) de fluide corporel sur le champ de test (128) de la bandelette de test optique (114) ;
d) la capture d'au moins une deuxième image d'au moins une partie du champ de test (128) de la bandelette de test optique (114) sur laquelle est appliqué l'échantillon (130) en utilisant la caméra (118) ;
e) la détermination d'au moins un élément d'informations d'admissibilité, dans lequel l'élément d'informations d'admissibilité indique une admissibilité au cas où la position du dispositif mobile (112) est sensiblement la même pour la capture de la première et de la deuxième image, dans lequel l'élément d'informations d'admissibilité est déterminé sur la base d'une ou des deux parmi les données de capteur de position et les données de position locale, dans lequel les données de position locale sont ou comprennent des informations spatiales se référant à une position d'au moins une caractéristique environnementale dans un champ de vision de la caméra, dans lequel la caractéristique environnementale est différente de la bandelette de test ou de parties de la bandelette de test et du dispositif mobile ou de parties de celui-ci ; et
f) si l'élément d'informations d'admissibilité indique une admissibilité, la détermination d'une valeur de résultat de mesure analytique en utilisant la première et la deuxième image du champ de test (128) de la bandelette de test optique (114)
dans lequel l'étape e) comprend la récupération d'au moins un premier élément d'informations de position comprenant les informations sur la position du dispositif mobile lors de la capture de la première image à l'étape b) et la récupération d'au moins un deuxième élément d'informations de position comprenant les informations sur la position du dispositif mobile lors de la capture de la deuxième image à l'étape d),
dans lequel l'étape e) comprend en outre la comparaison du premier et du deuxième élément d'informations de position entre eux, et dans lequel l'élément d'informations d'admissibilité indique une admissibilité au cas où le deuxième élément d'informations de position est, au moins dans une plage de tolérance prédéterminée, identique au premier élément d'informations de position.

2. Procédé selon la revendication précédente, dans lequel le procédé comprend en outre
g) si l'élément d'informations d'admissibilité indique une inadmissibilité, la réalisation d'un ou des deux parmi :
- l'affichage d'un message d'erreur sur l'écran (120) du dispositif mobile (112) ; et
- l'interruption du procédé de réalisation d'une mesure analytique.

3. Procédé selon l'une quelconque des revendications précédentes, dans lequel la caméra (118) est une caméra avant du dispositif mobile (112), dans lequel la caméra avant et l'au moins un écran (120) du dispositif mobile (112) sont tous deux positionnés sur une partie avant du dispositif mobile (112).

4. Procédé selon l'une quelconque des revendications précédentes, dans lequel dans les étapes b) et d) le dispositif mobile (112) est positionné dans une position fixe par une ou les deux parmi :
- l'utilisation d'un support pour le dispositif mobile (112) ; et
- la mise en place du dispositif mobile (112) sur une surface fixe.

5. Procédé selon la revendication précédente, dans lequel la surface fixe est une surface choisie dans le groupe constitué par : une surface horizontale, telle qu'une table, une surface d'assise, un plancher ou une étagère ; une surface inclinée ou en pente ; une surface plane ; une surface irrégulière.

6. Procédé selon l'une quelconque des revendications précédentes, dans lequel lors de la capture de l'au moins une première image à l'étape b), le champ de test (128) de la bandelette de test optique (114) est éclairé par l'utilisation de l'écran (120) du dispositif mobile (112), dans lequel lors de la capture de l'au moins une deuxième image à l'étape d), le champ de test (128) de la bandelette de test optique (114) est éclairé en utilisant l'écran (120) du dispositif mobile (112).

7. Procédé selon l'une quelconque des revendications précédentes, dans lequel le procédé comprend en outre :
h) la fourniture d'indications sur où positionner la bandelette de test optique (114) pour capturer la première et/ou la deuxième image en utilisant le dispositif mobile (112).

8. Procédé selon la revendication précédente, dans lequel l'indication est fournie en utilisant l'écran (120) du dispositif mobile (112), dans lequel l'indication sur où positionner la bandelette de test optique (114) de l'étape h) comprend la superposition d'une image en direct de la caméra (118) sur l'écran (120) du dispositif mobile (112) avec un guidage visuel.

9. Procédé selon l'une quelconque des revendications précédentes, dans lequel l'étape d) comprend la capture d'une pluralité de deuxièmes images, dans lequel le procédé comprend la surveillance de cinétiques de réaction en utilisant la pluralité de deuxièmes images.

10. Programme informatique comprenant des instructions qui, lorsque le programme est exécuté par un dispositif mobile (112) ayant une caméra (118), amènent le dispositif mobile (112) à effectuer au moins les étapes e) et f), et éventuellement les étapes b) et d), du procédé selon l'une quelconque des revendications précédentes.

11. Support de stockage lisible par ordinateur, spécifiquement un support de stockage non transitoire, comprenant des instructions qui, lorsqu'elles sont exécutées par un dispositif mobile (112) ayant une caméra (118), amènent le dispositif mobile (112) à effectuer au moins les étapes e) et f), et éventuellement les étapes b) et/ou d), du procédé selon l'une quelconque des revendications de procédé précédentes.

12. Dispositif mobile (112) permettant la réalisation d'une mesure analytique, le dispositif mobile (112) ayant au moins une caméra (118), au moins un écran (120) et un capteur de position (124), le dispositif mobile (112) étant configuré pour réaliser au moins les étapes e) et f), et éventuellement les étapes b) et/ou d), du procédé de réalisation d'une mesure analytique selon l'une quelconque des revendications précédentes se référant à un procédé de réalisation d'une mesure analytique.

13. Kit (110) permettant la réalisation d'une mesure analytique, le kit (110) comprenant :
- au moins un dispositif mobile (112) selon l'une quelconque des revendications précédentes se référant à un dispositif mobile (112) ; et
- au moins une bandelette de test optique (114) ayant au moins un champ de test (128).
